# EUROPÄISCHE PATENTSCHRIFT

(11) **EP 3 586 132 B1**
(45) Veröffentlichungstag und Bekanntmachung des Hinweises auf die Patenterteilung: **07.06.2023**
(21) Anmeldenummer: 18707335.8
(22) Anmeldetag: 22.02.2018
(51) Int. Cl.: G01N 33/50, G01N 33/569, C07K 16/28, A61K 39/395

(54) **MEDIKAMENT ZUR MALIGNOMBEHANDLUNG**
MEDICAMENT FOR MALIGNANT TUMOR TREATMENT
MÉDICAMENT DESTINÉ AU TRAITEMENT DE MALIGNITÉ

(30) Priorität: 27.02.2017 DE 102017001875
(43) Veröffentlichungstag der Anmeldung: 01.01.2020
(73) Patentinhaber: Intellexon GmbH, 82343 Pöcking (DE)
(72) Erfinder: WÜRFEL, Wolfgang, 85235 Sixtnitgern (DE)
(74) Vertreter: Samson & Partner Patentanwälte mbB
(86) Internationale Anmeldenummer: PCT/EP2018/054339
(87) Internationale Veröffentlichungsnummer: WO 2018/153956

(56) Entgegenhaltungen:
- WO-A1-2016/032334
- WO-A1-2016/041945
- WO-A1-2016/062851
- WO-A2-2006/070286
- EMILY M. MCWILLIAMS ET AL: "Therapeutic CD94/NKG2A blockade improves natural killer cell dysfunction in chronic lymphocytic leukemia", ONCOIMMUNOLOGY, Bd. 5, Nr. 10, 9. September 2016 (2016-09-09), Seite e1226720, XP055465259, DOI: 10.1080/2162402X.2016.1226720
- Emily M Mcwilliams ET AL: "Targeting the Tumor Evasion Interaction of NKG2A and Its Ligand HLA-E Increases Natural-Killer Cell Activity in Chronic Lymphocytic Leukemia", Blood, 2. Oktober 2016 (2016-10-02), Seiten 1289-1291, XP055465598, DOI: 10.1080/2162402X.2016.1226720 Gefunden im Internet: URL:http://www.bloodjournal.org/content/12 6/23/1289?sso-checked=true [gefunden am 2018-04-09]
- FARAH KETROUSSI ET AL: "Lymphocyte Cell-Cycle Inhibition by HLA-G Is Mediated by Phosphatase SHP-2 and Acts on the mTOR Pathway", PLOS ONE, Bd. 6, Nr. 8, 24. August 2011 (2011-08-24) , Seite e22776, XP55465313, DOI: 10.1371/journal.pone.0022776
- GRZYWACZ B ET AL: "Use of natural killer cells as immunotherapy for leukaemia", BEST PRACTICE & RESEARCH CLINICAL HAEMATOLOGY, ELSEVIER, AMSTERDAM, NL, Bd. 21, Nr. 3, 1. September 2008 (2008-09-01), Seiten 467-483, XP025404486, ISSN: 1521-6926, DOI: 10.1016/J.BEHA.2008.07.008 [gefunden am 2008-09-12]

## Beschreibung

### Gebiet der Erfindung

Die vorliegende Erfindung betrifft das Gebiet der Bereitstellung von Medikamenten zur Behandlung von Malignomen.

### Hintergrund der Erfindung

Neben den klassischen Therapien zur Behandlung von malignen Tumorerkrankungen, z.B. Resektion, Chemotherapie und Strahlentherapie, werden zunehmend aktive oder passive Immuntherapien angewandt, basierend auf der Verabreichung von Impfstoffen zur Auslösung einer Immunantwort beziehungsweise von Antikörper(fragmente)n zur Bindung an das Malignom. Medikamente zur Behandlung von Malignomen sollen hochselektiv sein und keine Resistenzen entstehen lassen.

Nichtsdestotrotz stehen den Tumorerkrankungen Mechanismen zur Verfügung, einer Immunantwort zu entgehen. Ein solcher sogenannter Escape-Mechanismus basiert auf dem MHC (Major Histocompatibility Complex), insbesondere mit seinen HLA-Gruppen (Human Leucocyte Antigen), der dem zellulären Dialog beim Menschen dient. Die Abkürzung HLA kann in der Literatur kodierende Gene oder von diesen exprimierte Proteine bezeichnen. Der im Folgenden verwendete Begriff HLA-Gruppen soll hierbei insbesondere die von den Genen auf der Zelloberfläche exprimierten Oberflächenproteine bezeichnen.

Im Allgemeinen können HLA-Gruppen beispielsweise in folgende vier Klassen eingeteilt werden:
(i) HLA-Gruppen A, B und C (MHC-I), die im Wesentlichen alle adulten und somatischen Zellen identifizieren;
(ii) HLA-Gruppen D (DR, DP, DQ usw.; MHC-II), die eine wichtige Rolle bei immunkompetenten Zellen bzw. in der Antigenpräsentierung spielen;
(iii) HLA-Gruppen E, F und G, die embryonale Zellen, insbesondere an der sogenannten Invasionsfront, identifizieren;
(iv) HLA-Gruppen H und folgende, die sogenannten Pseudo-Gene.

Malignomzellen können typische embryonale HLA-Gruppen (d.h. HLA-E, HLA-F und/oder HLA-G) auf ihrer Oberfläche exprimieren. Embryonale HLA-Gruppen können dazu beitragen, dass Malignomzellen dem Angriff der unspezifischen und/oder spezifischen Immunabwehr des eigenen Organismus entgehen. Durch die Expression dieser typischen HLA-Gruppen auf der Oberfläche der Zellen werden diese in die Lage versetzt, entsprechende Rezeptoren auf immunkompetenten Zellen zu aktivieren. In der Regel handelt es sich um Rezeptoren, die nach Aktivierung die Funktion dieser immunkompetenten Zellen hemmen, beispielsweise die Killer-Immunglobulinähnlichen Rezeptoren (KIR) auf den natürlichen Killerzellen oder die Leukozyten-Immunglobulinähnlichen Rezeptoren (LILR) auf den Lymphozyten.

So verhindern insbesondere die Antigene HLA-E, -F und -G auf den embryonalen (vornehmlich auf plazentaren bzw. trophoblastären) Zellen, dass das Immunsystem der Mutter die Zellen angreift. Auf diese Weise können Embryonen der Immunantwort entgehen. Dieser Escape-Mechanismus stellt das Rückgrat der immunologischen Steuerung der Schwangerschaft dar. Eine Abstoßungsreaktion unterbleibt und der genetisch halbfremde (väterlicher Fremdanteil 50 %), bzw. fremde (bei Eizell- oder Embryonenspenden oder Leihmutterschaften 100%) Embryo kann ausgetragen werden.

Maligne Tumoren der verschiedensten Gewebe sind in der Lage, sich diesen embryonalen Escape-Mechanismus nutzbar zu machen, womit sie die Immunabwehr unterbinden oder vermindern. Hierdurch sind sie auch in der Lage, manche therapeutische Strategien zu konterkarieren, also die auf einem Angriff fußende Strategie zu hemmen. Aus diesem Grund kann es vorteilhaft sein, das Immunsystem einzubeziehen um das Malignom zu behandeln.

Aus WO 2006/070286 sind Antikörper bekannt, die NKG2a-Rezeptoren auf NK-Zellen binden sollen, ohne die Zellen zu inhibieren.

Aus der EP 2 561 890 A1 sind Herstellungsverfahren für Antitumor-Medikamente bekannt, bei denen nach Bestimmen eines Expressionsmusters einer Malignomzelle dort exprimierte embryonale HLA-Gruppen maskiert oder zerstört werden. Die maskierten oder zerstörten HLA-Gruppen können das Immunsystem nicht mehr hemmen, sodass mit einem Angriff der immunkompetenten Zellen zu rechnen ist. Hierfür ist eine Maskierung möglichst aller Malignomzellen notwendig. Die Maskierung der HLA-Gruppen kann jedoch auch Auswirkungen auf andere, nicht-maligne Gewebe haben, die ebenfalls embryonale HLA-Gruppen exprimieren.

Vor diesem Hintergrund ist es eine Aufgabe der Erfindung Verfahren zur Bereitstellung von Antikörpern zur Behandlung von Malignomen zur Verfügung zu stellen.

### Kurzbeschreibung der Erfindung

Diese Aufgabe wird gelöst durch das Verfahren gemäß dem unabhängigen Anspruch 1. Die abhängigen Ansprüche beschreiben bevorzugte Ausführungsformen.

Ein erfindungsgemäßes Verfahren zur Bereitstellung eines Medikaments zur Behandlung eines Malignoms unter Einbeziehung des jeweils individuell zugehörigen Immunsystems umfasst das Ermitteln der individuellen Kommunikationsstruktur zwischen dem Malignom und dem Immunsystem sowie das Bereitstellen von Antikörpern.

Zum Ermitteln der individuellen Kommunikationsstruktur zwischen dem Malignom und dem Immunsystem wird sowohl wenigstens ein Expressionsmuster von an dem Malignom befindlichen embryonalen HLA-Gruppen bestimmt, als auch wenigstens ein an/in immunkompetenten Zellen des Immunsystems vorhandener Rezeptorstatus oder -typ bestimmt. Selbst histopathologisch identisch klassifizierte Tumore oder Metastasen können von einer Lokalisation zu einer anderen Lokalisation interindividuell oder intraindividuell unterschiedliche Expressionsmuster aufweisen. Therapien, z.B. Gabe von Chemotherapeutika oder Hormonantagonisten, können die Expressionsmuster weiter beeinflussen. Eine Bestimmung der individuellen Expressionsmuster geht auf diese Unterschiede ein.

Das Bestimmen wenigstens eines Rezeptorstatus, insbesondere Rezeptortyps, bezieht sich auf wenigstens einen Rezeptortyp, der in der Lage ist, zumindest einen Teil des Expressionsmusters als Ligand zu binden und, basierend auf dieser Bindung, eine inhibitorische Wirkung auf die immunkompetenten Zellen auszuüben. Bei diesen Rezeptortypen des jeweiligen Rezeptorstatus handelt es sich um eine Klasse von Oberflächen- oder Transmembranproteinen, die von den immunkompetenten Zellen auf ihrer Oberfläche exprimiert werden und mittels Signaltransduktion Signalpfade in Gang setzen.

Inhibitorische Wirkung soll hier den immun-modulatorischen Effekt bezeichnen, welcher die zytotoxische Aktivität der immunkompetenten Zellen vermindert oder verhindert. Dieser Signalweg kann zum Beispiel über das Immunrezeptor-Tyrosin-basierte inhibitorische Motiv (ITIM), d.h. zytoplasmatische Phosphorylierung, ausgelöst werden.

Das Bereitstellen von Antikörpern bezieht sich auf Antikörper des Typs, die einerseits als Liganden an den wenigstens einen bestimmten Rezeptortyp spezifisch binden, jedoch die zugehörige immunkompetente Zelle nicht inhibieren und andererseits den Rezeptor so blockieren oder maskieren, dass der wenigstens eine Teil des Expressionsmusters dort nicht oder nur noch mit geringerer Wirkung binden kann. Durch Maskierung oder Blockade des Rezeptors und somit Hinderung der Bindung des Expressionsmusters von embryonalen HLA-Gruppen an diesen Rezeptor kann deren inhibitorische Wirkung vermieden werden.

Im Allgemeinen sind die erfindungsgemäß bereitgestellten Antikörper in der Lage, durch Bindung an den Rezeptor keine inhibitorische Wirkung auf die immunkompetenten Zellen auszuüben, d.h. keine intrinsische Aktivität aufzuweisen. Im Gegensatz hierzu üben die Expressionsmuster von embryonalen HLA-Gruppen der Malignomzelle bei Bindung an den Rezeptor eine inhibitorische Wirkung auf die immunkompetenten Zellen aus.

In manchen Ausführungsformen umfasst das Bestimmen des Expressionsmusters eine Prüfung ob die embryonalen HLA-Gruppen präsent sind oder nicht. Vorzugsweise umfasst das Bestimmen des Expressionsmusters ferner die quantitative Bestimmung eines Expressionslevels. Beispielsweise können Expressionsmuster oder Expressionslevel durch bekannte Verfahren, wie RNA-Sequenzierung, DNA-Mikroarrays, quantitative PCR (quantitative Polymerase Chain Reaction), expression profiling, SAGE (serial analysis of gene expression, serielle Genexpressionsanalyse), etc. bestimmt werden.

Der Begriff Malignomzelle umfasst auch Metastasenzellen des Primärmalignoms. Das erfindungsgemäße Verfahren wird für vorzugsweise mehrere, besonders verzugsweise für alle Metastasen individuell durchgeführt, um auf etwaige individuelle Unterschiede der Metastasen, insbesondere deren individuelle Expressionsmuster von deren embryonalen HLA-Gruppen, einzugehen.

Vorzugsweise ist der wenigstens eine Rezeptortyp typischerweise als Transmembranprotein an der Oberfläche der immunkompetenten Zellen exprimiert. In manchen Ausführungsformen umfasst der wenigstens eine Rezeptortyp einen oder mehrere der folgenden: KIR-Rezeptoren (Killer-Immunglobulinähnlichen Rezeptoren), NKG2-Rezeptoren, LIL-R-Rezeptoren (Leukozyten-immunglobulinähnliche Rezeptoren).

Manche NKG2-Rezeptoren, insbesondere NKG2 A, NKG2 B, NKG2 C, NKG2 D, NKG2 E und NKG2 F, können beispielsweise HLA-E binden. Manche LIL-Rezeptoren, insbesondere LIL B1, LIL B2 und LIL B4, können beispielsweise HLA-F binden. KIR 2DL3 oder LIL A2 können beispielsweise HLA-G binden.

Die bereitgestellten Antikörper sind des Typs, an wenigstens einen Rezeptortyp zu binden. So binden beispielsweise die als Anti-KIR 2DS1 bezeichneten Antikörper als Liganden an Rezeptoren des Typs KIR 2DS1. Die als Anti-KIR 2DS4 bezeichneten Antikörper können an Rezeptoren des Typs KIR 2DS4 binden.

In manchen Ausführungsformen kann das Bestimmen des Rezeptortyps auch berücksichtigen, welche Rezeptoren bei dem Individuum angelegt sind. Die Überprüfung, welche Rezeptoren bei dem Individuum angelegt sind, kann beispielsweise mittels Gen- oder Expressionsanalyse durchgeführt werden. Falls ein bestimmter Rezeptortyp bei dem Individuum nicht vorhanden ist, so ist eine Verabreichung von Antikörpern gegen diesen Rezeptortyp nicht indiziert.

In manchen Ausführungsformen umfassen die immunkompetenten Zellen NK-Zellen (natürliche Killerzellen) und/oder Lymphozyten.

In manchen Ausführungsformen umfasst das Verfahren ferner das Bereitstellen von aktivierenden Antikörpern des Typs, die nach Bindung an einen Rezeptor der immunkompetenten Zellen eine aktivierende Wirkung auf diese auslösen.

In manchen Ausführungsformen kann das Verfahren ferner ein Ermitteln, ob HLA-C überexprimiert ist, umfassen. Bei Überexpression von HLA-C kann dessen Expressionsmuster und ein zweiter Rezeptortyp für dieses Expressionsmuster bestimmt werden; nämlich ein solcher, der in der Lage ist, zumindest einen Teil des HLA-C-Expressionsmusters als Ligand zu binden und - basierend auf dieser Bindung - eine aktivierende Wirkung auf immunkompetente Zellen auszuüben. Beispielsweise kann es sich bei dem zweiten Rezeptortyp um einen Rezeptortyp aus der Gruppe der KIR-DS-Rezeptoren handeln, welche eine aktivierende Wirkung auf NK-Zellen ausüben können. Beispiele von aktivierenden Rezeptoren, die an HLA-C binden sind KIR 2DS1, KIR 2DS2 oder KIR 2DS4. Derartige Rezeptoren des Typs KIR-DS können immunkompetente Zellen aktivieren, sodass diese beispielsweise wachstumsaktive und das Tumorwachstum fördernde Substanzen, wie Zytokine oder Wachstumsfaktoren, produzieren.

Alternativ kann der zweite Rezeptortyp in der Lage sein, zumindest einen Teil des HLA-C-Expressionsmusters als Ligand zu binden, und basierend auf dieser Bindung eine inhibitorische Wirkung auf immunkompetente Zellen auszuüben. Beispielsweise kann es sich bei dem zweiten Rezeptortyp um einen Rezeptortyp aus der Gruppe der KIR-DL-Rezeptoren handeln, welche eine inhibitorische Wirkung auf NK-Zellen ausüben können. Beispiele von hemmenden Rezeptoren, die an HLA-C binden sind KIR 2DL1, KIR 2DL2, KIR 2DL3 oder KIR 3DL3.

Ausgehend von dem zweiten Rezeptortyp können zweite Antikörper des Typs bereitgestellt werden, die einerseits an den zweiten Rezeptortyp spezifisch binden, jedoch die zugehörige immunkompetente Zelle nicht inhibieren oder aktivieren, gleichzeitig den zweiten Rezeptor so blockieren oder maskieren, dass der wenigstens eine Teil des HLA-C-Expressionsmusters dort nicht oder nur noch mit geringerer Wirkung binden kann.

Des Weiteren offenbart die Beschreibung Antikörper , welche durch ein erfindungsgemäßes Verfahren bereitgestellt wurden; außerdem die Verwendung von erfindungsgemäßen Antikörpern als Medikament bei der Behandlung eines Malignoms.

In manchen Ausführungsformen umfasst die offenbarte Verwendung das Blockieren oder Maskieren von Rezeptoren des wenigstens einen bestimmten Rezeptortyps auf immunkompetenten Zellen mittels der Antikörper. Bei einer derartigen Verwendung der Antikörper wird die Bindung von auf Malignomzellen exprimierten embryonalen HLA-Gruppen an die Rezeptoren des wenigstens einen bestimmten Rezeptortyps auf den immunkompetenten Zellen verhindert oder vermindert. Vorzugsweise weisen die Antikörper eine hohe Affinität zu dem Rezeptortyp auf, insbesondere vergleichbar zu, größer als oder wesentlich größer als die Affinität der embryonalen HLA-Gruppen der Malignomzellen.

Vorzugsweise ist die Affinität groß genug, um ein Abdiffundieren und/oder kompetitives Verdrängen der Antikörper zu verhindern.

Insbesondere kann das Blockieren oder Maskieren in vivo, z.B. im Organismus des Patienten, erfolgen. In manchen Ausführungsformen kann die Verwendung der Antikörper lokal oder systemisch erfolgen. Lokale Verwendung umfasst beispielsweise die Injektion in den oder in die Nähe des Malignoms. Systemische Verwendung umfasst beispielsweise die Darreichung auf einem der folgenden Wege: oral, nasal, sublingual, rektal, subkutan, intravenös, perkutan etc.

Alternativ zu einer Blockade in vivo kann das Blockieren oder Maskieren in vitro mit anschließender Transfusion der immunkompetenten Zellen erfolgen, beispielsweise um systemische Nebenwirkungen zu vermeiden. Für eine Blockade in vitro können immunkompetente Zellen entnommen, den Antikörpern ausgesetzt und nach erfolgter Maskierung zurücktransfundiert werden.

In manchen Ausführungsformen einer erfindungsgemäßen Verwendung stammen die Zelle, deren Expressionsmuster bestimmt wurde, und/oder die immunkompetenten Zellen, deren Rezeptoren spezifisch durch die Antikörper gebunden werden, von ein und demselben Patienten, beispielsweise um systemische Nebenwirkungen zu vermeiden. Solche Ausführungsformen können insbesondere bevorzugt sein, falls die inter-individuelle Variation der Expression von embryonalen HLA-Gruppen groß ist.

In manchen Ausführungsformen können die immunkompetenten Zellen, deren Rezeptoren spezifisch durch die Antikörper gebunden werden, von einem Spender stammen. Bei dem Spender kann es sich um einen Dritten handeln, der nicht an dem Malignom erkrankt ist. Die immunkompetenten Zellen können beispielsweise nach erfolgter Blockade oder Maskierung der Rezeptoren injiziert werden und verwendet werden um das Malignom, dessen Expression von embryonalen HLA-Gruppen bestimmt wurde, zu behandeln.

### Kurzbeschreibung der Zeichnungen

In der folgenden Beschreibung von Ausführungsbeispielen der Erfindung wird auf die beigefügten Zeichnungen Bezug genommen, die zeigen:
FIG. 1 ein Flussdiagramm eines Verfahrens gemäß einem Ausführungsbeispiel,
FIG. 2 eine schematische Darstellung von drei Malignomzellen verschiedener Individuen, an denen ein Verfahren gemäß einem Ausführungsbeispiel durchgeführt werden kann,
FIG. 3 eine schematische Darstellung einer Malignomzelle und einer immunkompetenten Zelle, mit Bindung zwischen einer embryonalen HLA-Gruppe und einem Rezeptor der immunkompetenten Zelle,
FIG. 4 eine schematische Darstellung einer Malignomzelle und einer immunkompetenten Zelle, an denen ein Verfahren gemäß einem Ausführungsbeispiel durchgeführt werden kann,
FIG. 5 eine schematische Darstellung einer Malignomzelle und einer immunkompetenten Zelle, an denen ein Verfahren gemäß einem Ausführungsbeispiel durchgeführt werden kann,
FIG. 6 eine schematische Darstellung von einer Malignomzelle und zwei immunkompetenten Zellen, an denen ein Verfahren gemäß einem Ausführungsbeispiel durchgeführt werden kann,
FIG. 7 eine schematische Darstellung einer Malignomzelle und einer immunkompetenten Zelle, mit Maskierung eines Rezeptors der immunkompetenten Zelle durch einen Antikörper gemäß einem Ausführungsbeispiel.

### Beschreibung von bevorzugten Ausführungsbespielen

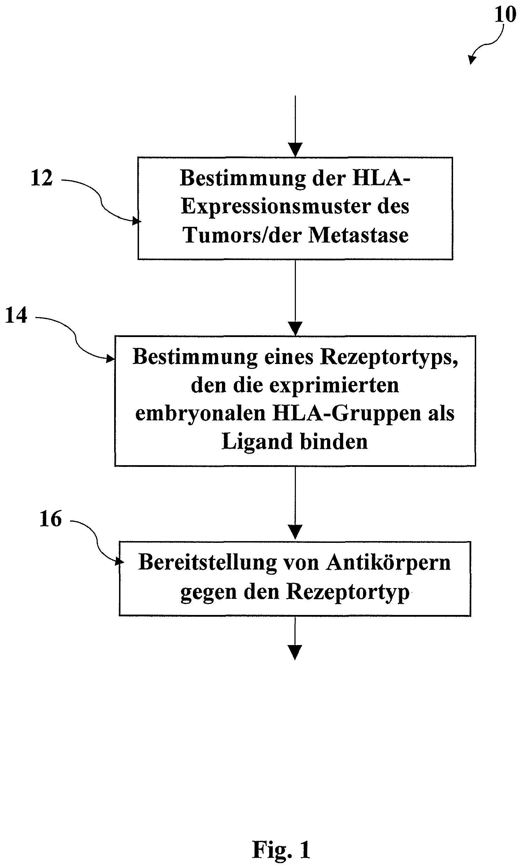

FIG. 1 zeigt ein Flussdiagramm eines Verfahrens 10 zur Bereitstellung eines Medikaments. Das Verfahren 10 umfasst ein Bestimmen 12 eines Expressionsmusters, ein Bestimmen 14 wenigstens eines Rezeptortyps und ein Bereitstellen 16 von Antikörpern.

Beim Bestimmen 12 des Expressionsmusters werden die Expressionen von an dem Malignom befindlichen embryonalen HLA-Gruppen bestimmt.

Beim Bestimmen 14 wenigstens eines Rezeptortyps, wird ein Rezeptortyp bestimmt, der in der Lage ist, zumindest einen Teil des Expressionsmusters der embryonalen HLA-Gruppen als Ligand zu binden und basierend auf dieser Bindung eine inhibitorische Wirkung auf die immunkompetenten Zellen auszuüben.

Die in Schritt 16 bereitgestellten Antikörper sind des Typs, die als Liganden an den wenigstens einen bestimmten Rezeptortyp der immunkompetenten Zellen spezifisch binden und den Rezeptor so blockieren oder maskieren, dass der wenigstens eine Teil des Expressionsmusters der embryonalen HLA-Gruppen dort nicht oder nur noch mit geringerer Wirkung als der Antikörper binden kann, selbst aber die zugehörige immunkompetente Zelle nicht inhibierten kann.

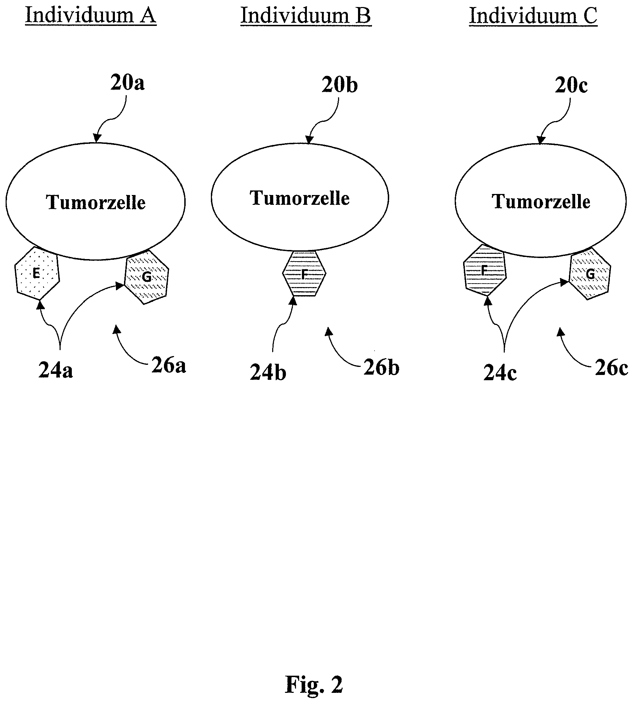

**FIG. 2** zeigt eine schematische Darstellung von drei Malignomzellen 20a, 20b, 20c verschiedener Individuen A, B und C, an denen ein erfindungsgemäßes Verfahren durchgeführt werden kann. Insbesondere kann anhand jeder der drei dargestellten Malignomzellen 20a, 20b, 20c ein jeweiliges Expressionsmuster bestimmt werden, insbesondere ein für das jeweilige Individuum oder das jeweilige Malignom individuelles Expressionsmuster. Die drei Malignomzellen 20a, 20b, 20c unterscheiden sich in ihrer individuellen Kommunikationsstruktur mit dem Immunsystem.

Eine erste Malignomzelle 20a ist dem Individuum A entnommen worden. Diese Malignomzelle 20a kann als Teil einer Gewebeprobe eines Malignoms des Individuum A entnommen worden sein. Die Malignomzelle 20a weist an ihrer Oberfläche eine Vielzahl von Membranproteinen auf (nicht dargestellt). Einige dieser Membranproteine gehören zu den embryonalen HLA-Gruppen. Die Malignomzelle 20a weist insbesondere embryonale HLA-Gruppen 24a des Typs HLA-E und des Typs HLA-G (nicht jedoch des Typs HLA-F) auf. Ein Expressionsmuster 26a der durch die Malignomzelle 20a exprimierten embryonalen HLA-Gruppen 24a kann also bestimmt werden.

Eine zweite Malignomzelle 20b, die einem Individuum B entnommen worden ist, kann in den von ihr exprimierten embryonale HLA-Gruppen 24b von den durch die erste Malignomzelle 20a exprimierten übereinstimmen, teilweise abweichen oder vollständig abweichen. Im dargestellten Fall exprimiert die zweite Malignomzelle 20b embryonale HLA-Gruppen 24b des Typs HLA-F. Es kann also auch ein Expressionsmuster 26b der durch die Malignomzelle 20b exprimierten embryonalen HLA-Gruppen 24b bestimmt werden.

Auch für eine dritte Malignomzelle 20c, die einem Individuum C entnommen worden ist, kann ein Expressionsmuster 26c von embryonalen HLA-Gruppen 24c, die durch die Malignomzelle 20c exprimiert sind, bestimmt werden. Im dargestellten Fall umfasst das Expressionsmuster 26c die embryonalen HLA-Gruppen 24c des Typs HLA-F und des Typs HLA-G.

Ausgehend von einem bestimmten Expressionsmuster wird wenigstens ein Rezeptortyp bestimmt, der in der Lage ist, eine der im Expressionsmuster enthaltenen embryonalen HLA-Gruppen als Ligand zu binden und basierend auf dieser Bindung eine inhibitorische Wirkung auf immunkompetente Zellen auszuüben.

Bei Zustandekommen einer Rezeptor-Ligand-Bindung, wobei der Rezeptor auf einer ersten Zelle (z.B. einer immunkompetenten Zelle) exprimiert ist und der Ligand auf einer zweiten Zelle (z.B. einer Malignomzelle) exprimiert sein kann, findet ein zellulärer Dialog statt und dieses Zusammenwirken kann beispielsweise eine aktivierende oder inhibitorische Wirkung entfalten. Auch die Identifikation von Zellen basiert auf solchen Rezeptor-Ligand-Bindungen.

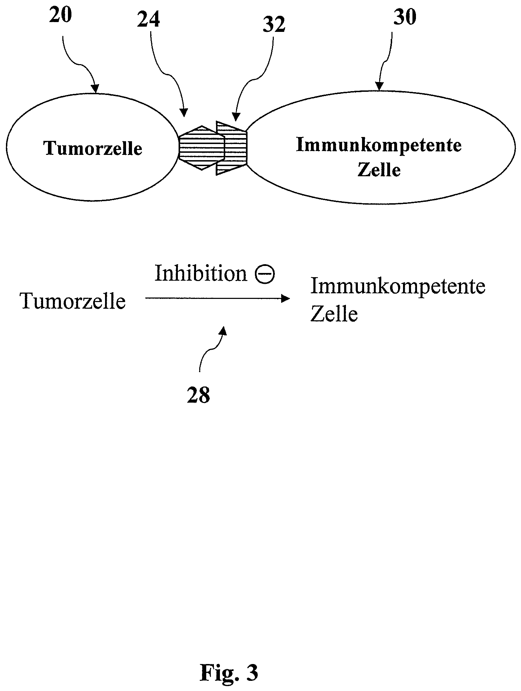

**FIG. 3** zeigt eine schematische Darstellung einer Malignomzelle 20 und einer immunkompetenten Zelle 30, mit Bindung zwischen einer embryonalen HLA-Gruppe 24 der Malignomzelle 20 und einem Rezeptor 32 der immunkompetenten Zelle 30. Basierend auf der dargestellten Bindung der embryonalen HLA-Gruppe 24 übt die Malignomzelle 20 eine inhibitorische Wirkung 28 auf die immunkompetente Zelle 30 aus. Diese Wechselwirkung zwischen dem Expressionsmuster der Malignomzelle 20 und dem Rezeptor der immunkompetenten Zelle ist charakteristisch für eine individuelle Kommunikationsstruktur zwischen dem Malignom und dem Immunsystem. Diese inhibitorische Wirkung hat zur Folge, dass eine Immunantwort der immunkompetenten Zelle 30 im Wesentlichen unterbleibt.

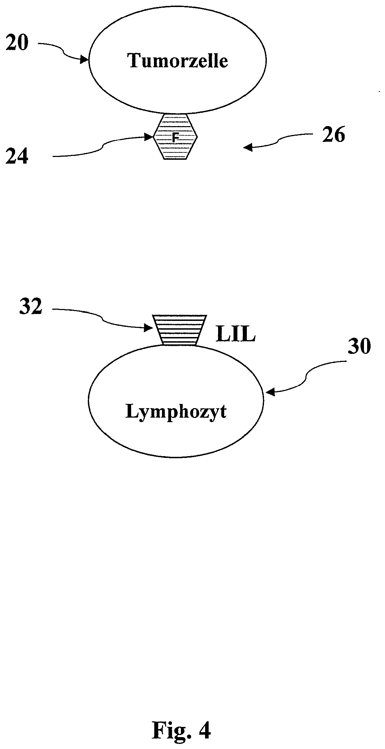

**FIG. 4** zeigt eine schematische Darstellung einer Malignomzelle 20 und einer immunkompetenten Zelle 30, an denen ein erfindungsgemäßes Verfahren durchgeführt werden kann. Insbesondere verdeutlicht dieses Ausführungsbeispiel, wie anhand eines bestimmten Expressionsmusters wenigstens ein Rezeptortyp bestimmt werden kann. Diese Bestimmung trägt dazu bei, die individuelle Kommunikationsstruktur zwischen dem Malignom und dem Immunsystem zu ermitteln.

So kann an der Malignomzelle 20 ein Expressionsmuster 26 von embryonalen HLA-Gruppen bestimmt werden, das HLA-Gruppen des Typs HLA-F umfasst.

Basierend auf dem bestimmten Expressionsmuster 26 wird wenigstens ein Rezeptortyp 32 bestimmt, der in der Lage ist, eine der exprimierten embryonalen HLA-Gruppen, nämlich die HLA-Gruppen 24 vom Typ HLA-F, als Ligand zu binden, und basierend auf der Bindung der embryonalen HLA-Gruppe 24 eine inhibitorische Wirkung auf die immunkompetente Zelle 30 auszuüben. Bei der immunkompetenten Zelle 30 handelt es sich um einen Lymphozyten. Ein Rezeptortyp 32, der die oben genannten Voraussetzungen erfüllt, ist LILR B1 (leucocyte immunoglobulin-like receptor B1).

Ohne Durchführung eines erfindungsgemäßen Verfahrens könnte die Malignomzelle 20 mittels der HLA-Gruppe 24 an den inhibitorischen Rezeptor 32 des Lymphozyten 30 binden und somit eine Hemmung des Immunsystems hervorrufen. Dies stellt einen Escape-Mechanismus der Malignomzelle dar, um dem Angriff des Immunsystems zu entgehen.

In einem erfindungsgemäßen Verfahren kann basierend auf dem bestimmten Expressionsmuster 26 und dem bestimmten Rezeptortyp 32 ein Antikörper bereitgestellt werden, der diesen Escape-Mechanismus unterbindet. Die Bereitstellung der Antikörper kann beispielsweise gemäß bekannten Herstellungs- oder Isolierverfahren, wie der Hybridom-Technik, durchgeführt werden.

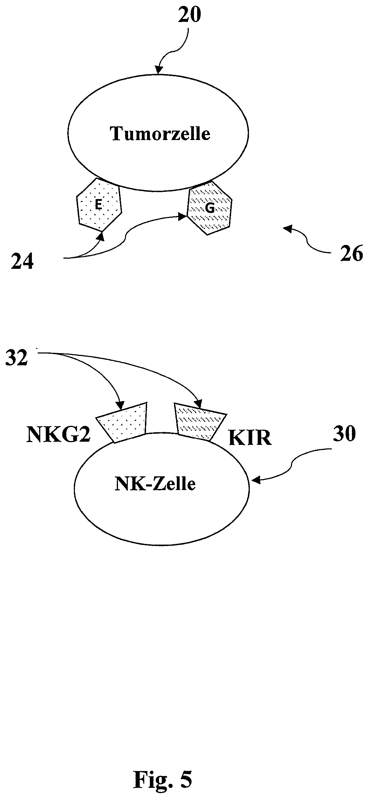

**FIG. 5** zeigt eine schematische Darstellung einer weiteren Malignomzelle 20 und eines Lymphozyt 30, an denen ein Verfahren zur Bereitstellung eines Medikaments durchgeführt werden kann. An der Malignomzelle 20 kann wiederum ein Expressionsmuster 26 von durch die Malignomzelle exprimierten embryonalen HLA-Gruppen bestimmt werden. Das Expressionsmuster 26 umfasst HLA-Gruppen 24 vom Typ HLA-E und HLA-G. Insbesondere verdeutlicht dieses Ausführungsbeispiel, wie anhand eines bestimmten Expressionsmusters mehrere Rezeptortypen bestimmt werden können, die für die individuelle Kommunikationsstruktur zwischen dem Malignom und dem Immunsystem charakteristisch sind.

Basierend auf dem Expressionsmuster 26 werden zwei Rezeptortypen 32 bestimmt, nämlich Rezeptoren NKG2 und KIR. Diese beiden Rezeptortypen 32 sind in der Lage, eine der exprimierten embryonalen HLA-Gruppen 24 als Ligand zu binden, und basierend auf der Bindung der embryonalen HLA-Gruppe eine inhibitorische Wirkung auf die immunkompetente Zelle 30 auszuüben. So sind die NKG2-Rezeptoren in der Lage, HLA-Gruppen vom Typ HLA-E als Ligand zu binden und basierend auf der Bindung der HLA-E-Gruppe eine inhibitorische Wirkung auf den Lymphozyt 30 auszuüben. Ferner sind die KIR-Rezeptoren in der Lage, HLA-Gruppen vom Typ HLA-G als Ligand zu binden und basierend auf der Bindung der HLA-G-Gruppe eine inhibitorische Wirkung auf den Lymphozyt 30 auszuüben.

Falls, wie in dem dargestellten Beispiel, mehrere Rezeptortypen bestimmt werden, so können diese mehreren Rezeptortypen oder zumindest ein Teil davon blockiert oder maskiert werden, wobei eine gewisse Hierarchie der Blockade beachtet werden kann. So gilt beispielsweise der Rezeptor vom Typ KIR 2DL4 als besonders wichtig für die hemmende Wirkung. Zur Vermeidung von Nebenwirkung kann also eine Beschränkung auf die wichtigsten Rezeptortypen bevorzugt sein, insbesondere bei eine Maskierung in vivo, um systemische Nebenwirkungen zu vermeiden.

In manchen Ausführungsformen können einzelne Rezeptortypen Schritt für Schritt blockiert werden, z.B. falls eine relative Einordnung der hemmenden Wirkungen von mehreren Rezeptortypen im Einzelfall noch nicht bekannt ist. Bei ausbleibender oder unzureichender Immunantwort können in weiteren Schritten ein oder mehrere weitere oder andere Rezeptortypen blockiert werden. Ein solches Vorgehen kann insbesondere bei einer in-vivo-Maskierung bevorzugt sein, um systemische Nebenwirkungen zu vermeiden. Somit lässt sich eine fein dosierte Steuerung der Maskierung und der Antwort des Immunsystems vornehmen.

In anderen Ausführungsformen können, z.B. falls eine relative Einordnung der hemmenden Wirkungen von mehreren Rezeptortypen noch nicht bekannt sein sollte, alle oder zumindest eine große Zahl von Rezeptortypen gleichzeitig maskiert werden. Ein derartiges Vorgehen kann insbesondere bei einer in-vitro-Maskierung bevorzugt sein, unter anderem weil dort systemische Nebenwirkungen durch die Antikörper im Allgemeinen weniger wahrscheinlich sind. Zudem werden bei einer in-vitro-Maskierung typischerweise nur ein Teil aller immunkompetenten Zellen entnommen, sodass der Einfluss einer Maskierung auf das Immunsystem in dieser Hinsicht begrenzt ist.

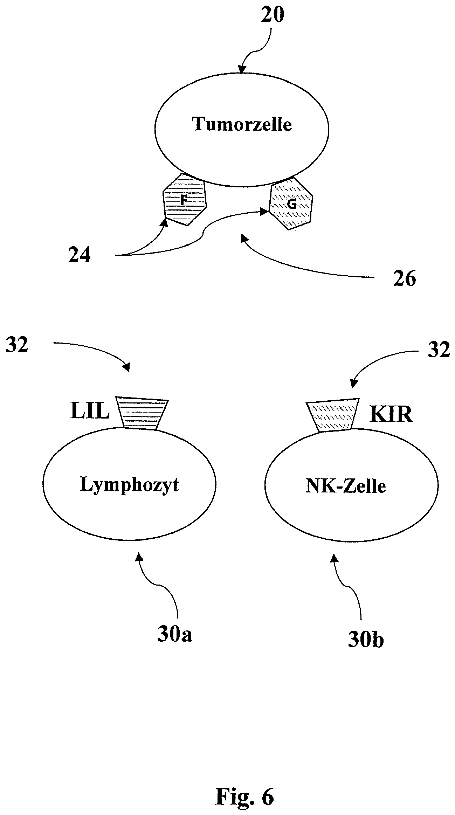

**FIG. 6** zeigt eine schematische Darstellung von einer Malignomzelle 20 und zwei immunkompetenten Zellen, nämlich einem Lymphozyt 30a und eine NK-Zelle 30b, an denen ein Verfahren zur Bereitstellung eines Medikaments durchgeführt werden kann. An der Malignomzelle 20 kann wiederum ein Expressionsmuster 26 von durch die Malignomzelle exprimierten embryonalen HLA-Gruppen bestimmt werden. Das Expressionsmuster 26 umfasst HLA-Gruppen 24 vom Typ HLA-F und HLA-G. Insbesondere verdeutlicht dieses Ausführungsbeispiel, dass die bestimmten Rezeptortypen inhibitorische Wirkung auf verschiedene immunkompetente Zellen, wie Lymphozyten und NK-Zellen, haben können. Es wird somit die individuelle Kommunikationsstruktur zwischen Malignom und dem Immunsystem ermittelt.

Basierend auf den beiden exprimierten HLA-Gruppen werden zwei Rezeptortypen 32 bestimmt, nämlich LIL-R (leucocyte immunoglobulin-like receptor) und KIR (killer cell immunoglobulin-like receptor). Die LIL-Rezeptoren sind in der Lage, HLA-Gruppen vom Typ HLA-F als Ligand zu binden und basierend auf der Bindung der HLA-F-Gruppe eine inhibitorische Wirkung auf den Lymphozyt 30a auszuüben. Ferner sind die KIR-Rezeptoren in der Lage, HLA-Gruppen vom Typ HLA-G als Ligand zu binden und basierend auf der Bindung der HLA-G-Gruppe eine inhibitorische Wirkung auf die NK-Zellen 30b auszuüben. Somit sind die beiden Rezeptortypen 32 in der Lage, eine der exprimierten embryonalen HLA-Gruppen 24 als Ligand zu binden, und basierend auf der Bindung der embryonalen HLA-Gruppe eine inhibitorische Wirkung auf verschiedene immunkompetente Zellen 30a, 30b auszuüben.

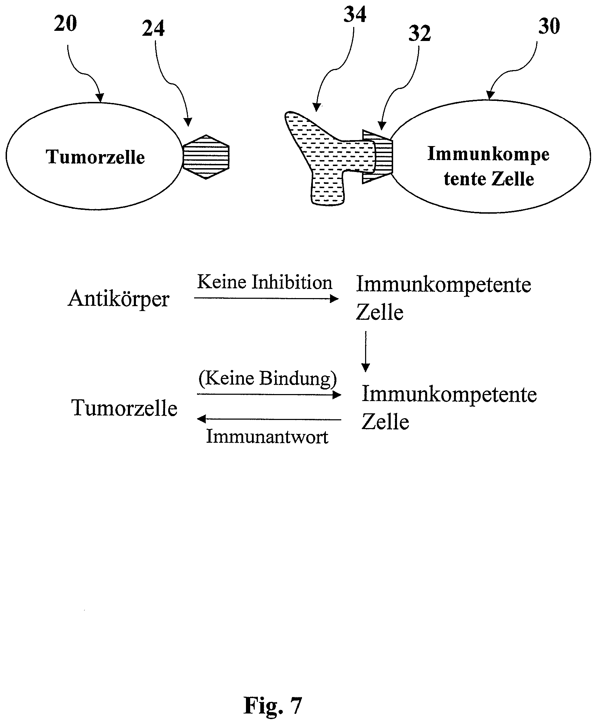

**FIG. 7** zeigt eine schematische Darstellung einer Malignomzelle 20 mit einer HLA-Gruppe 24 und einer immunkompetenten Zelle 30 mit einem Rezeptor 32. Ferner ist eine erfindungsgemäßer Antikörper 34 dargestellt. Insbesondere verdeutlicht dieses Ausführungsbeispiel, wie ein erfindungsgemäßer Antikörper einen Rezeptor blockieren oder maskieren kann.

Der Rezeptortyp 32 ist zwar grundsätzlich in der Lage, die embryonalen HLA-Gruppe 24 der Malignomzelle 20 als Ligand zu binden, und basierend auf der Bindung der embryonalen HLA-Gruppe 24 eine inhibitorische Wirkung auf die immunkompetente Zelle 30 auszuüben. Jedoch ist der Rezeptor 32 der immunkompetenten Zelle 30 durch einen Antikörper 34 maskiert.

Der Antikörper 34 ist des Typs, der als Ligand an den Rezeptor 32 spezifisch bindet und hierdurch einerseits den Rezeptor 32 so blockiert oder maskiert, dass die embryonale HLA-Gruppe 24 an den Rezeptor 32 nicht oder nur noch mit geringerer Wirkung binden kann, andererseits keine inhibitorische Wirkung auf die zugehörige immunkompetente Zelle ausübt. Das Prinzip eines Liganden, der an einen Rezeptor bindet und diesen nicht aktiviert, ist aus der Pharmakologie bekannt, beispielsweise zu den in der Reproduktionsmedizin genutzten Gonadotropin-Releasing-Hormon (GnRH) - Rezeptoren. GnRH-Antagonisten sind Beispiele von Liganden, die an einen Rezeptor binden und diesen nicht aktivieren.

Ohne den Antikörper 34 könnte die Malignomzelle 20 mittels der HLA-Gruppe 24 an den inhibitorischen Rezeptor 32 der immunkompetenten Zelle 30 binden und somit eine Hemmung des Immunsystems hervorrufen. Dies stellt einen Escape-Mechanismus der Malignomzelle dar, um dem Immunsystem zu entgehen.

In Anwesenheit des Antikörpers 24 kann der inhibitorische Rezeptor 32 maskiert werden und der Escape-Mechanismus unterbunden werden. Die Immunantwort der immunkompetenten Zelle 30 ist nicht inhibiert. Somit kann der Antikörper der Behandlung des Malignoms unter Einbeziehung des jeweils individuell zugehörigen Immunsystems dienen.

Der für eine Maskierung des inhibitorischen Rezeptors 32 verwendete Antikörper kann in eine Bibliothek von humanisierten Antikörpern gegen inhibitorische Rezeptortypen von immunkompetenten Zellen aufgenommen werden. Dies bedeutet, dass die Vielfalt der Bibliothek von der Anzahl unterschiedlicher inhibitorischer Rezeptortypen abhängt. Falls die Bindung eines weiteren Expressionsmusters, beispielsweise von HLA-Pseudogenen, an diese Rezeptortypen (deren Antikörper bereits Teil der Bibliothek sind) blockiert oder maskiert werden soll, so ist keine Erweiterung der Bibliothek (z.B. durch Herstellung von neuen, humanisierten Antikörpern) notwendig, sondern es kann für die Bereitstellung von Antikörpern auf die bestehende Bibliothek zugegriffen werden.

## Patentansprüche

1. Verfahren zur Bereitstellung von Antikörpern zur Behandlung eines Malignoms unter Einbeziehung des jeweils individuell zugehörigen Immunsystems, umfassend
(a) Ermitteln der individuellen Kommunikationsstruktur zwischen dem Malignom und dem Immunsystem, beinhaltend
- Bestimmen wenigstens eines Expressionsmusters von mehreren an dem Malignom befindlichen embryonalen HLA-Gruppen, wobei die embryonalen HLA-Gruppen HLA-E, HLA-F und HLA-G umfassen und wobei das Bestimmen des Expressionsmusters ein Prüfen, ob die embryonalen HLA-Gruppen exprimiert sind, umfasst, und
- Bestimmen, basierend auf dem bestimmten Expressionsmuster, wenigstens eines an/in immunkompetenten Zellen des Immunsystems vorhandenen Rezeptortyps, der in der Lage ist, zumindest einen Teil der exprimierten embryonalen HLA-Gruppen als Ligand zu binden und basierend auf dieser Bindung eine inhibitorische Wirkung auf die immunkompetenten Zellen auszuüben und
(b) Bereitstellen von Antikörpern, des Typs, die
- als Liganden an den wenigstens einen bestimmten Rezeptortyp spezifisch binden und hierdurch den Rezeptor so blockieren oder maskieren, dass der wenigstens eine Teil der exprimierten embryonalen HLA-Gruppen dort nicht oder nur noch mit geringerer Wirkung binden kann,
- selbst aber die zugehörige immunkompetente Zelle nicht inhibieren.

2. Verfahren gemäß Anspruch 1, wobei der wenigstens eine Rezeptortyp einen oder mehrere der folgenden umfasst: KIR-Rezeptoren, NKG2-Rezeptoren, LIL-R-Rezeptoren.

3. Verfahren gemäß einem der vorstehenden Ansprüche, zusätzlich umfassend:
- Bereitstellen von aktivierenden Antikörpern, vom Typ, die nach Bindung an einen Rezeptor einer immunkompetenten Zelle eine aktivierende Wirkung auf die immunkompetente Zelle auslösen.

4. Verfahren gemäß einem der vorstehenden Ansprüche, umfassend:
(a') Ermitteln ob HLA-C überexprimiert ist, und bei Überexpression von HLA-C
- Bestimmen eines HLA-C-Expressionsmusters von HLA-C-Gruppen,
- Bestimmen eines zweiten Rezeptortyps, der in der Lage ist, zumindest einen Teil des HLA-C-Expressionsmusters als Ligand zu binden, und basierend auf dieser Bindung eine inhibitorische oder aktivierende Wirkung auf immunkompetente Zellen auszuüben und
(b') Bereitstellen von zweiten Antikörpern, des Typs, die
- an den zweiten Rezeptortyp spezifisch binden und hierdurch den zweiten Rezeptor so blockieren oder maskieren, dass der wenigstens eine Teil des HLA-C-Expressionsmusters dort nicht oder nur noch mit geringerer Wirkung binden kann,
- selbst aber die zugehörige immunkompetente Zelle nicht inhibieren oder aktivieren.

## Claims

1. A method for preparing antibodies for the treatment of a malignant tumor with inclusion of the particular individual, associated immune system, comprising
(a) determining the individual communication structure between the malignant tumor and the immune system, comprising
- determining at least one expression pattern of multiple embryonic HLA groups present on the malignant tumor, wherein the embryonic HLA groups comprise HLA-E, HLA-F and HLA-G and wherein the determining of the expression pattern comprises checking whether the embryonic HLA groups are expressed, and
- determining, based on the determined expression pattern, at least one receptor type present on/in immunocompetent cells of the immune system which type is capable of bonding at least a part of the expressed embryonic HLA groups as ligand and, based on this bond, of exerting an inhibitory effect on the immunocompetent cells, and
(b) providing antibodies of the type which
- specifically bond as ligands to the at least one determined receptor type and as a result block or mask the receptor in such a manner that the at least one part of the expressed HLA groups cannot bond there or can only bond there with a lesser effect,
- but do not inhibit the associated immunocompetent cell themselves.

2. The method according to Claim 1, wherein the at least one receptor type comprises one or more of the following: KIR receptors, NKG2 receptors, LIL-R receptors.

3. The method according to one of the previous claims, additionally comprising:
- providing of activating antibodies of the type which initiate an activating effect on the immunocompetent cell after having bonded to a receptor of an immunocompetent cell.

4. The method according to one of the previous claims, comprising:
(a') determining whether HLA-C is overexpressed and in the case of an overexpression of HLA-C
- determining an HLA-C expression pattern of HLA-C groups,
- determining a second receptor type which is capable of bonding at least a part of the HLA-C expression pattern as ligand and, based on this bond, of exerting an inhibitory or activating effect on immunocompetent cells, and
(b') providing second antibodies of the type which
- specifically bond to the second receptor type and as a result block or mask to the second receptor in such a manner that the at least one part of the HLA-C expression pattern cannot bond there or can only bond there with a lesser effect,
- but do not inhibit or activate the associated immunocompetent cell themselves.

## Revendications

1. Procédé pour préparer des anticorps pour le traitement d'une tumeur maligne avec inclusion du système immunitaire associé d'un individu particulier, comprenant
(a) la détermination de la structure de communication de l'individu entre la tumeur maligne et le système immunitaire, comprenant
- la détermination d'au moins un profil d'expression de groupes HLA embryonnaires multiples présents sur la tumeur maligne, lesquels groupes HLA embryonnaires comprennent HLA-E, HLA-F et HLA-G, et laquelle détermination du profil d'expression comprend la vérification que les groupes HLA embryonnaires sont ou non exprimés, et
- la détermination, sur la base du profil d'expression déterminé, d'au moins un type de récepteur présent sur/dans des cellules immunocompétentes du système immunitaire, lequel type est capable de se lier à au moins une partie des groupes HLA embryonnaires exprimés sous la forme d'un ligand et, sur la base de cette liaison, d'exercer un effet inhibiteur sur les cellules immunocompétentes, et
(b) l'obtention d'anticorps d'un type tel
- qu'ils se lient spécifiquement sous la forme de ligands à l'au moins un type de récepteur déterminé et, en résultat, bloquent ou masquent le récepteur de telle manière que l'au moins une partie des groupes HLA exprimés ne puissent pas s'y lier ou puissent s'y lier uniquement avec un effet moindre,
- mais qui n'inhibent pas les cellules immunocompétentes associées elles-mêmes.

2. Procédé selon la revendication 1, dans lequel l'au moins un type de récepteur comprend un ou plusieurs des suivants : les récepteurs KIR, les récepteurs NKG2, les récepteurs LIL-R.

3. Procédé selon l'une des revendications précédentes, comprenant de plus :
- l'obtention d'anticorps activés d'un type tel qu'ils initient un effet d'activation sur la cellule immunocompétente après avoir été liés à un récepteur d'une cellule immunocompétente.

4. Procédé selon l'une des revendications précédentes, comprenant :
(a') la détermination que HLA-C est ou non surexprimé et, dans le cas d'une surexpression de HLA-C
- la détermination d'un profil d'expression de HLA-C de groupes HLA-C,
- la détermination d'un deuxième type de récepteur qui est capable de se lier à au moins une partie du profil d'expression de HLA-C sous la forme d'un ligand et, sur la base de cette liaison, d'exercer un effet inhibiteur ou activateur sur des cellules immunocompétentes, et
(b') l'obtention de deuxièmes anticorps d'un type tel
- qu'ils se lient spécifiquement au deuxième type de récepteur et, en résultat, bloquent ou masquent le deuxième récepteur de telle manière que l'au moins une partie du profil d'expression de HLA-C ne puisse pas s'y lier ou puisse s'y lier uniquement avec un effet moindre,
- mais qui n'inhibent ou n'activent pas les cellules immunocompétentes associées elles-mêmes.
